# EUROPEAN PATENT SPECIFICATION

(11) **EP 3 020 422 B1**
(45) Date of publication and mention of the grant of the patent: **23.08.2017**
(21) Application number: 15190235.0
(22) Date of filing: 16.10.2015
(51) Int. Cl.: A61L 9/12

(54) **A MULTI-USE PACK WITH A PERFUMED CARTRIDGE**
MEHRFACHVERWENDUNGSPAKET MIT PARFÜMIERTER KARTUSCHE
BLOC MULTI-USAGE AVEC UNE CARTOUCHE PARFUMÉE

(30) Priority: 14.11.2014 IT RE20140095
(43) Date of publication of application: 18.05.2016
(73) Proprietor: RE.LE.VI. S.P.A., 46040 Rodigo (Mantova) (IT)
(72) Inventor: VENINI, CHERUBINO ROBERTO, 20121 Milano (IT)
(74) Representative: Corradini, Corrado

(56) References cited:
- WO-A1-2014/028682
- JP-A- 2004 182 312
- US-A1- 2007 262 166

## Description

The present invention relates to a multi-use packing system with a perfumed replacement cartridge, which can be made from card, cardboard or like materials, in a laminar and foldable form so that numerous permanent folds can be made.

The replacement cartridge is substantially flat and contains a volatile substance which disperses into the ambient air, in particular having a perfuming/refreshing function.

A pack and cartridge system is known, described in patent publication WO 2014/028682, which can be made from cardboard or a like material, which substantially comprises three walls connected two by two in a linear sequence, the two free ends whereof are then connected, so as to form, if arranged in an open configuration, an isosceles or equilateral triangle composed of a front wall, a rear wall and a horizontal wall which functions as a rest base; an opening is afforded on the front wall through which a central portion of the replacement cartridge is arranged, which is fixed detachably to the rear face of the first wall; in this configuration, the pack defines a display frame having a pleasant appearance, by means of which the cartridge is in a condition to dispense its perfuming substance into the surrounding environment, in particular for perfuming a house or office or an environment open to the public.

The pack can also take on a configuration in which all the walls are superposed on one another in a substantially flat shape; in this configuration, the pack can easily be transported, can be hung on display walls (for example in retail centres). Lastly, the replacement cartridge can be extracted from the pack and used with other means, for example with another display and dispensing structure.

Similar features are also in patent publication US 2007/262166 A1.

In JP 2004 182312 a packing system is shown having wings hinge-connected to the rear wall, foldably about a respective vertical fold edge, where the wings support the pouch in a stable erect position as a display frame.

An aim of the present invention is to improve the described packing system, especially for making the removable connection of the cartridge with the front wall simpler and more stable, and also for making the pack more secure with regard to possible attempts at tampering in the retail centres, with attempts being made to remove the replacement cartridge from the pack.

This and other aims are attained by the present invention as it is characterised in the claims.

Principally, the invention comprises:
a pack having three walls made of cardboard or a like material, hinge-connected two-by-two according to fold lines in end sides of the walls,
   - a replacement cartridge,
      wherein the pack comprises:
      a flat pouch formed by a first wall and by a second wall, which are superposed and fixed to one another in a substantially planar structure, the first wall has at least a first opening,
      the replacement cartridge is arranged removably in the pouch, at least partly exposed in the first opening,
      the second wall having a second opening which at least partly exposes a rear face of the replacement cartridge,
      the third wall being foldable with respect to the pouch;
   - in a first configuration in which it is laid on the pouch in a substantially planar state, in which the replacement cartridge cannot be removed from the pouch;and
   - in a second configuration in which the third wall is rotated distant from the pouch in a state that enables a user to remove the replacement cartridge from the pouch,
at least a wing being provided on the second wall or on the third wall, which wing is hinge-connected to the wall foldably about a respective vertical fold edge, where the wing supports the pouch in a stable erect position as a display frame, when, the third wall being in the first configuration, the wing is folded substantially by a right angle with respect to the pouch.

The invention is set out in the following with the aid of the accompanying figures, which illustrate some embodiments thereof by way of non-limiting example.
Figure 1 is a lateral front perspective view of a first embodiment of the packing system which includes the replacement cartridge, in a display frame.
Figure 2 is a lateral rear perspective view of the system of figure 1.
Figure 3 is a front view in vertical elevation of the system of figure 1.
Figure 4A is a rear front view in vertical elevation of the system of figure 1 in which the third wall is in the first configuration.
Figure 4B is the same view as in figure 4A, where the third wall is in the second configuration.
Figure 5 is a plan view of the cardboard structure which constitutes the pack, in the open and laid-out configuration.
Figure 6 is the section according to plane VI-VI of figure 3.
Figure 7A is the section according to plane VII-VII of figure 3, in which the wings of the third wall are in a coplanar position with respect to the third wall.
Figure 7B is the same section as figure 7A, in which the wings of the third wall are in a folded position perpendicular to the third wall.
Figure 7C is the same section as figure 7A, in which the third wall is in the second configuration, in which it enables extraction/introduction of the capsule in the pouch.
Figure 8 is a rear front view in vertical elevation of a second embodiment of the system in which the third wall is in the second configuration.
Figure 9A is a rear lateral perspective view of the system of figure 1, in which the wings 35 are folded with respect to the third wall in a non-complete way.
Figure 9B is the same as figure 9A, in which the wings 35 are completely folded with respect to the third wall so that the pouch is in a stable erect position as a display frame.
Figure 10 is a larger-scale detail of figure 9B.
Figure 11 is a front lateral perspective view of a third embodiment of the packing system which includes the replacement cartridge, in a display frame configuration.
Figure 12 is a rear lateral perspective view of the system of figure 11.

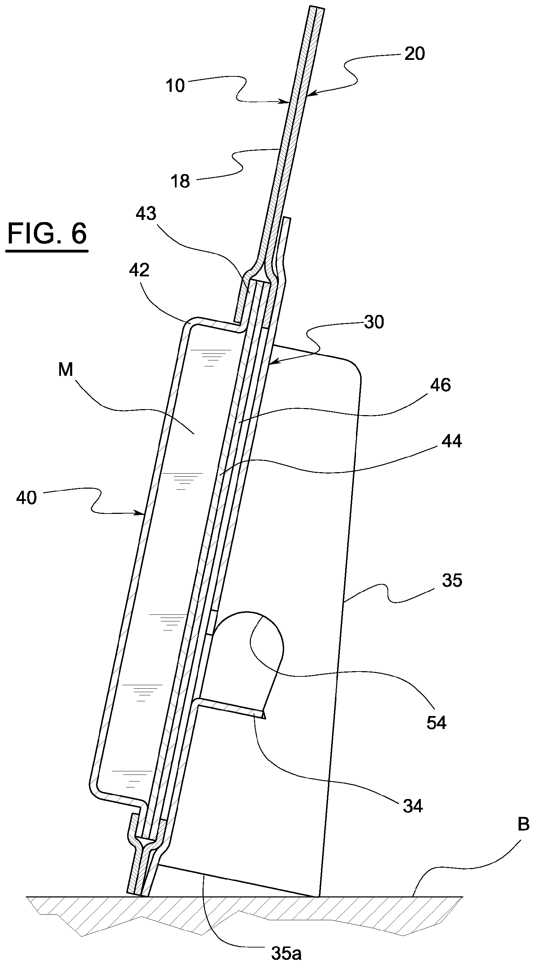

In figures 6, 7 and 10 the thicknesses of the sectioned elements are, in proportion, enlarged with respect to the other dimensions for the sake of clarity of illustration.

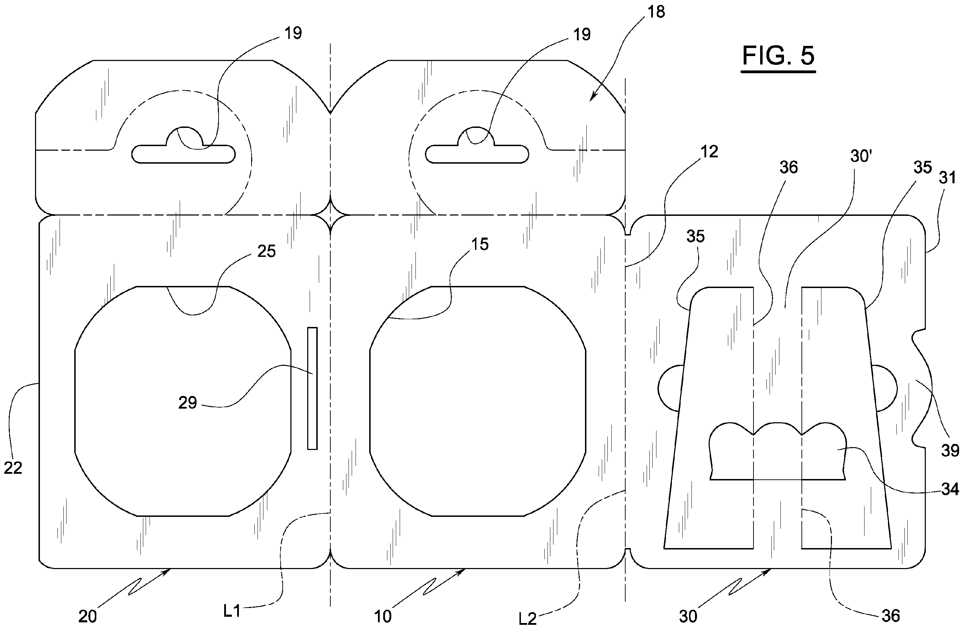

The system comprises a pack, typically made of cardboard, comprising three walls 10, 20, 30, hinge-connected two-by-two, at the folding edges L1 and L2 which are defined sides of the walls (see figure 5, which shows the cardboard structure in an open and laid-out configuration which, once folded, forms the pack).

The system further comprises a replacement cartridge 40, of known type, which encloses an active substance that permeates into the external environment. In particular the cartridge 40 is flat, i.e. the thickness thereof is several times smaller than the other two dimensions.

In the preferred embodiment, illustrated in the figures, the cartridge 40 comprises a blister 41 made of a synthetic heat-formed material having a central portion in the shape of a cup 42, and a flat peripheral external flange 43, which runs about the perimeter edge of the cup 42; a slim semipermeable wall 44 is fixed against the flange 43, which wall 44 delimits a chamber internally of the cup 43, in which the active substance M is contained.

In use, in a normal environment, the active substance M is in the liquid state and is retained internally of the cup 42 by the wall 44; however the wall 44 has a porosity that, while retaining the active substance in the liquid state, enables passage thereof gradually as it evaporates and passes to the gaseous state.

Lastly, a slim covering film 46, impermeable and separable, externally covers the wall 44 so as to prevent the evaporating active substance from being dispersed into the surrounding environment. The cover 46 has the aim of preventing dispersion of the active substance in the initial stage of the cartridge's life, from its composition up to the moment of first use thereof by a consumer; the user then removes the cover 46 when she or he wishes to put the container into the condition of dispensing the active substance into the air.

The first wall 10 and the second wall 20 are fixedly superposed on one another in a substantially planar structure, forming a flat pocket T; the exchange cartridge 40 is removably arranged in the pouch T and the cup portion 42 is exposed towards the outside through a first opening 15 afforded in the first wall 10.

The second wall 20 has a second opening 25 which at least partially externally opens towards the rear face of the replacement cartridge, where the semi-permeable wall 44 is arranged.

The third wall 30 is foldable with respect to the pouch T, in:
a first configuration in which it is laid on the pouch T in a substantially planar state, in which the replacement cartridge 40 cannot be removed from the pouch T;
a second configuration in which the third wall 30 is rotated distant from the pouch T in a non-planar state that enables a user to remove the replacement cartridge 40 from the pouch T.

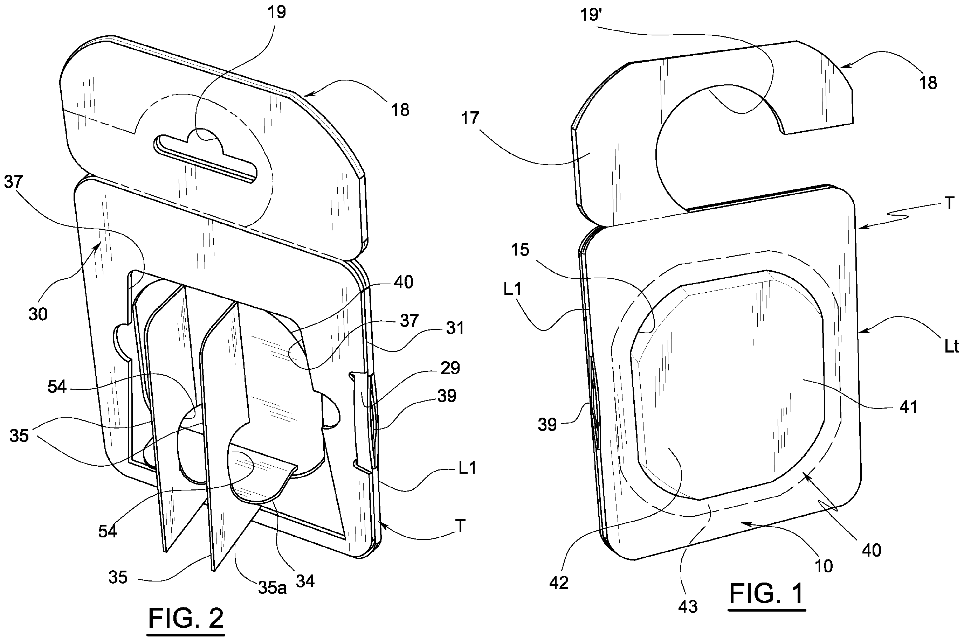

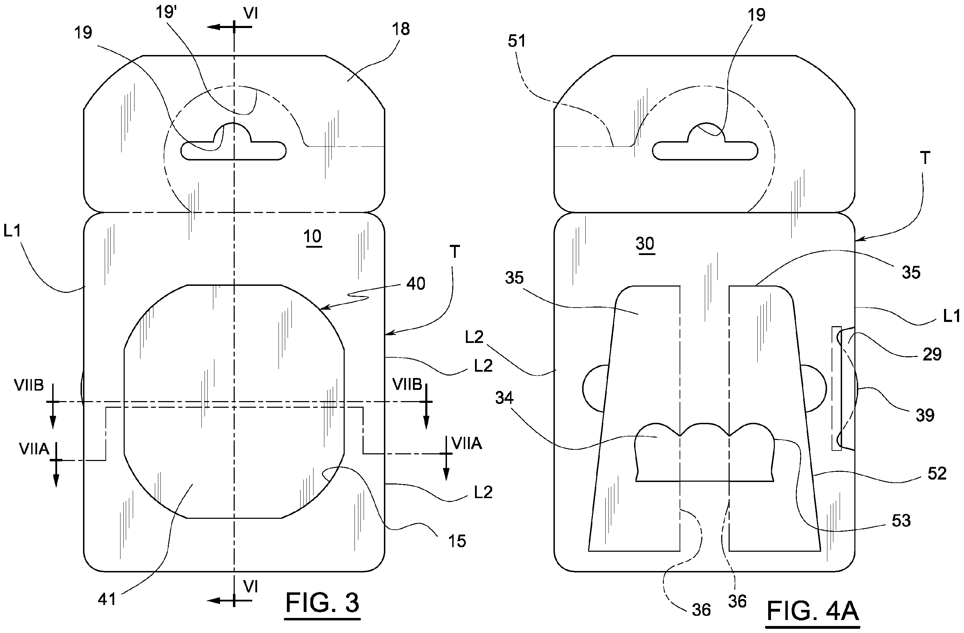

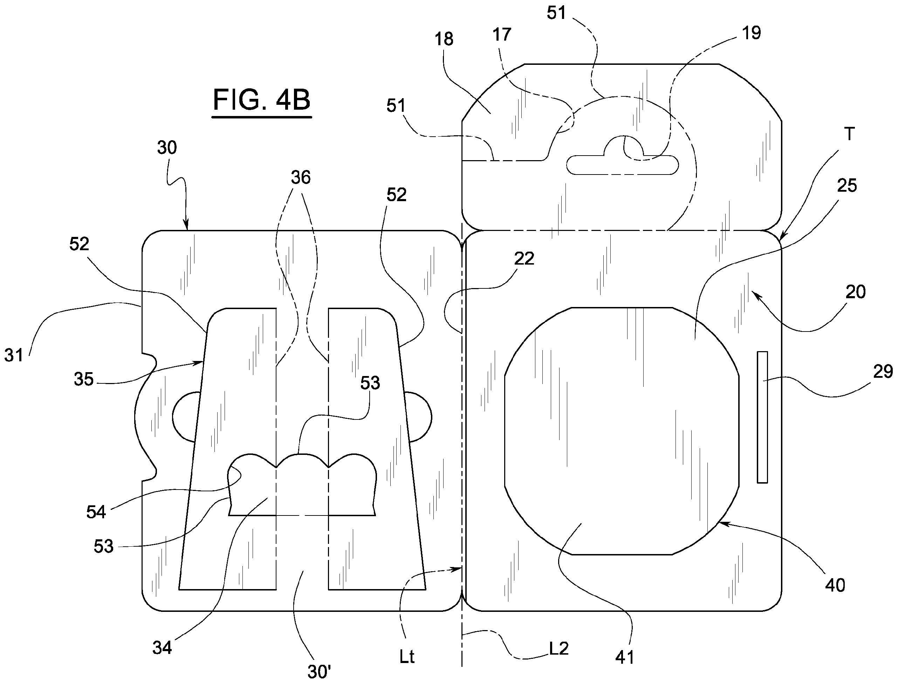

According to the embodiment illustrated in figures 1-7, the wall 10 and the wall 20 are hinge-connected foldably along a first foldable vertical edge L1 of the card, which defines a vertical side common to both the two walls 10 and 20; on the side opposite the edge L1, the second wall 20 comprises a vertical end side 22.

The first wall 10 and the third wall 30 are foldably hinge-connected to one another, along a second fold edge L2 which also defines a vertical side common to both the two walls 10 and 30. On the opposite side to the edge L2, the third wall 30 comprises a vertical end side 31.

Therefore: the first wall 10 is delimited between the two fold edges L1 and L2, the second wall 20 is delimited between the first edge L1 and the end side 22, and the third wall 30 is delimited between the second edge L2 and the end edge 31.

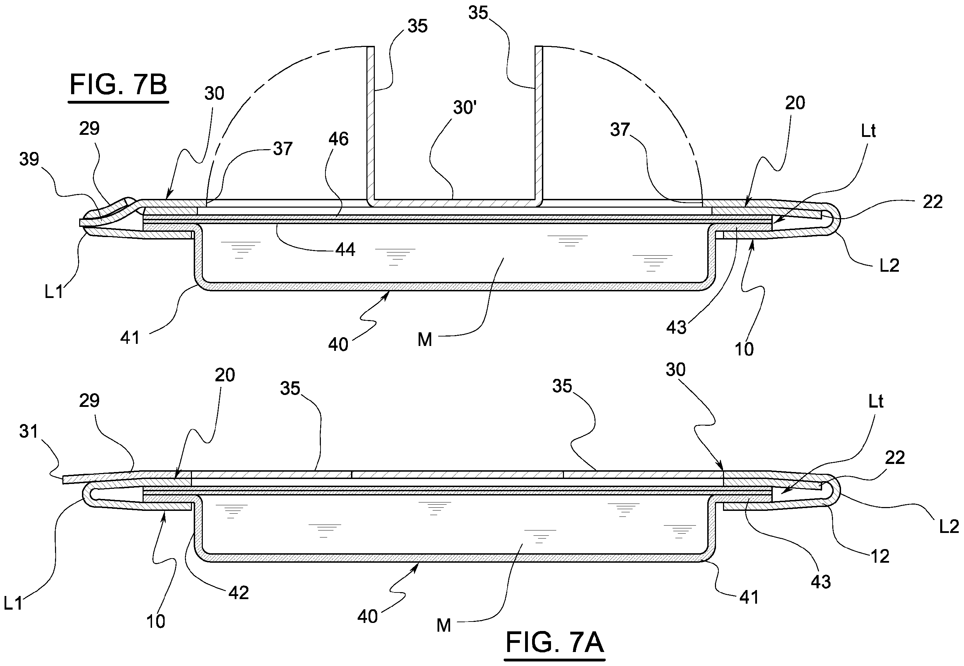

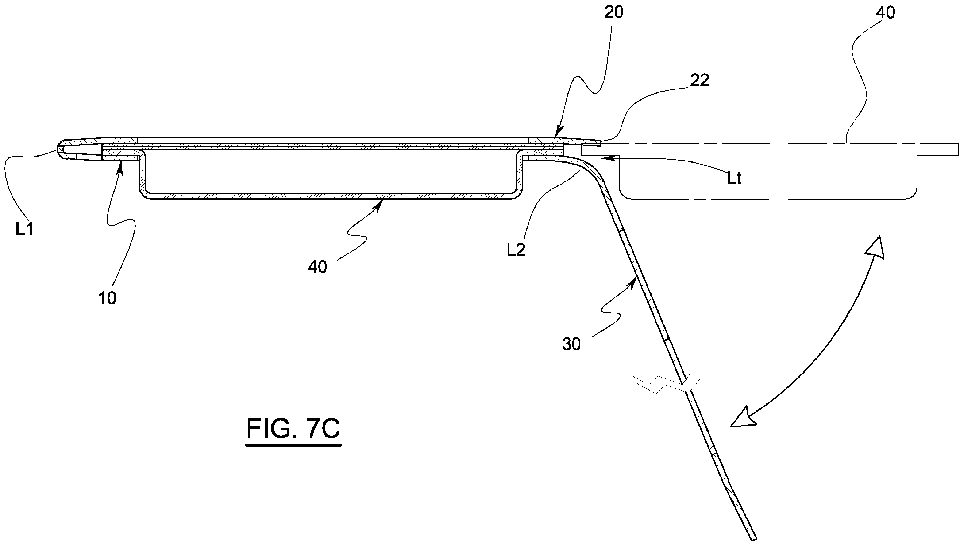

The sides of the two walls 10 and 20 both positioned at the second fold edge L2 and are reciprocally distanceable at the halfway line, while they are constrained at the two ends and define the access side Lt for introduction/extraction of the replacement cartridge internally of the pouch T; the third wall 30 covers each access side Lt of the pouch when it is in the first configuration (see figures 7A, 7B), and leaves the access side Lt accessible, when it is rotated away from the second wall 20, in the second configuration (see figure 7C).

Further, the third wall 30 at least partially superposes on the second wall 20 when it is in the first configuration, and is removably connectable thereto.

In detail, according to the embodiment illustrated in figures 1-7, the first wall 10 and the wall 20 substantially have the same dimensions; they are joined to one another along the fold line L1 common to both; the upper and lower horizontal sides of the two walls 10 and 20 are joined to one another along reciprocally-fixed horizontal lines (for example they are glued to one another); the central zones of the two walls 10, 20 are instead not constrained to one another; therefore the two walls 10 and 20 are solidly fixed to one another to define the pouch T, which has a flat envelope shape; the inside of the pouch is accessible through the second vertical side of the pouch, where the vertical sides L2 and 22 of the two walls 10, 20, which are separated from one another and define the access side Lt of the pouch T, through which the cartridge 40 can be extracted/introduced.

The cartridge inserted in the pouch T projects externally with the cup portion 42 thereof through the opening 15 on the wall 10; the opening 25 of the second wall 20 is afforded on the opening 15, which has the same shape and dimensions; the semipermeable wall 44 faces towards the external environment through the second opening 25.

In the first configuration, the third wall 30 is folded against the external surface of the second wall 20, which, as mentioned above, is folded permanently against the first wall 10. In this configuration the wall 30 closes, together with the first wall 10, the access side Lt of the pouch T, and prevents the exit of the cartridge 40 of the pouch T (see figures 7A and 7B).

In the embodiment illustrated in figures 1-7, to make the position of the third wall 30 stabler in adherence against the second wall 20, a projecting tongue 39 is provided on the free vertical side 31 of the third wall 30, i.e. the side opposite the side hinge-joined to the first wall 10, which projecting tongue 39 projects horizontally externally, while along the vertical end edge of the second wall 20, opposite the side where the access side Lt is located, a vertical strip 29 is included, in particular realized by two parallel and vertical cutting lines realised in the second wall 20 at the fold line L1, and a corresponding projection 39 (see figures 7A and 7B). The projection 39 is conformed such as to be inserted below the strip 29, realizing an effective constraint which keeps the third wall joined to the second sufficiently solidly, in reference to the forces in play (see figures 7A and 7B).

In order to extract/insert the cartridge 40 internally of the pouch T, the pack must be brought into a second configuration, in which the third wall 30 is rotated distant from the pouch T, in a state that frees the side Lt of the pouch; see figure 7C in which the third wall 30 has been rotated beyond 180 degrees (about 240 degrees in the figure).

When in the first configuration, the system has a substantially flat shape, which enables it to be hung on display walls, typically in retail sales outlets.

For this purpose, the two walls 10 and 20 (or alternatively one only thereof) exhibit an upper portion 18, which extends by a relatively large amount upwards with respect to the part of the pack that defines the pouch T. An opening 19 is afforded in this upper portion 18 which enables hanging the system to a hook.

Further, when in the first configuration, the system has a substantially flat shape, which enables it to be hung with the aim of dispensing the active substance into the surrounding environment; obviously, in order to enable dispensing of the active substance contained in the cartridge 40, it is first necessary to remove the impermeable cover 46 from the cartridge; this can be done after having extracted the cartridge from the pouch T through the access side Lt, then placing the cartridge free of the cover 46 in the pouch T.

In the illustrated embodiment in figures 1-7, a pre-cut line 51 is realised on the upper portion 18 which delimits a portion that, with a manual action by the user, can be removed, thus defining on the upper zone of the portion 18, externally of the line 51, an upper hook element 17 for hanging the system to a rear-view mirror of motor vehicles.

Further, the replacement cartridge can be extracted from the pack and used, alone, with other means, for example a display and dispensing frame.

The pouch T formed by the first two walls 10 and 20 can also be used as a display for supporting the cartridge 40 in an erect and suitable position for dispensing the active substance. For this purpose, one or two wings 35 are included on the second or third wall 20, 30, preferably on the third wall 30, which wings 35 are hinge-connected to the wall foldably about respective vertical fold lines 36. The wings 35 are conformed such as to enable the pouch to be erect, as a display frame, when it is folded substantially by a right-angle with respect to the pouch T.

The wings 35 are fashioned, by cutting lines made on the third wall 30 (or on the second wall 20) as portions of the wall, partly separated from the wall and hinge-connected foldably thereto, each about a respective vertical fold line 36.

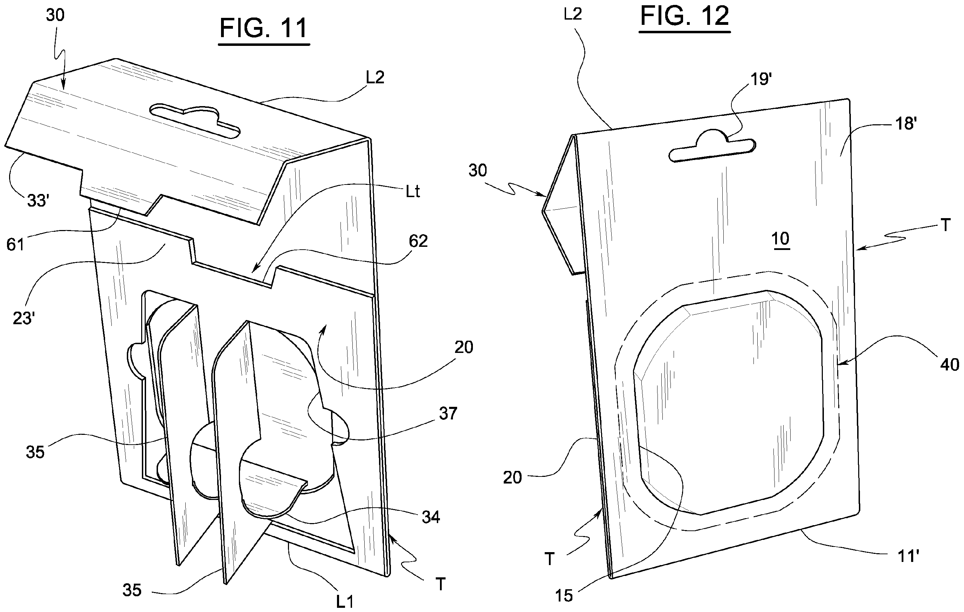

In detail, the wings 35 have an elongate shape in a vertical direction, and have a smaller base side 35a which is substantially horizontal and located at a short distance from the lower side of the wall 30 (or the wall 20 in figures 11 and 12); when the pack is in the closed configuration (first configuration), by folding the wings to a right-angle (or almost) with respect to the wall 30 (or 20), the pack can be arranged in an erect position by placing the lower side of the wall 30 (or 20) and the lower external end of the wings 35 restingly on a horizontal rest plane B (as illustrated in figure 6). In this configuration (third configuration) the system functions as a display case for the cartridge 40.

The wings 35 further define third openings 37 in the wall (or the wall 20) of which they are portions, when they are folded by a right angle with respect to the wall.

In the first embodiment illustrated in figures 1-7, the third wall 30 substantially covers the whole lower part of the second wall 20 and covers the opening 25; the wings are fashioned in the third wall 30, in such a position that the third openings 37 they form when rotated by 90 degrees with respect to the wall 30 superpose at least partially on the second opening 25 of the second wall 20, thus enabling the active substance to volatilize and exit from the pack through the openings 37.

In this embodiment, the fold lines 36 are arranged on the two vertical sides closest to one another, i.e. the sides located closest to the vertical median axis of the wall 30.

In the same zone where the wings 35 are fashioned, on the same wall 30, a small shelf 34 is created by cutting lines 53, which shelf 34 is folded along a horizontal fold axis and projects with a substantially horizontal lie; the cutting line 53 involves a portion of wings 35 and a portion of the central corridor 30' of the wall 30 delimited between the two wings 35. When the shelf 34 is folded perpendicularly to the wall 30, the cutting line 53 defines, on each wing 35, an opening 54 located at the fold line 36 of the wings; the shelf 34 engages in this opening 54, which is profiled so as to constrain, following an insertion with a slight forcing, the shelf to the wings 35, with the aim of maintaining the position of the wings sufficient stationary perpendicular to the wall 30.

In the first and second embodiments illustrated in the figures, two parallel vertical wings are preferably included, fashioned in the third wall 30, arranged symmetrically with respect to the vertical axis of the third wall 30; in the third embodiment (figures 11 and 12), the wings 35 are fashioned on the second wall 20.

The second embodiment (illustrated in figures from 8 to 10) differs from the preceding embodiment in that the wings 35, which are in this case too fashioned by means of cutting lines performed on the third wall 30, are portions of the wall, partially separated from the wall 30 and foldably hinge-connected to the wall 30, each about a respective vertical fold edge 36.

However, the fold lines 36 are arranged close to the two vertical end sides 31 and L2 of the wall 30, i.e. relatively distant from the median vertical axis of the wall 30.

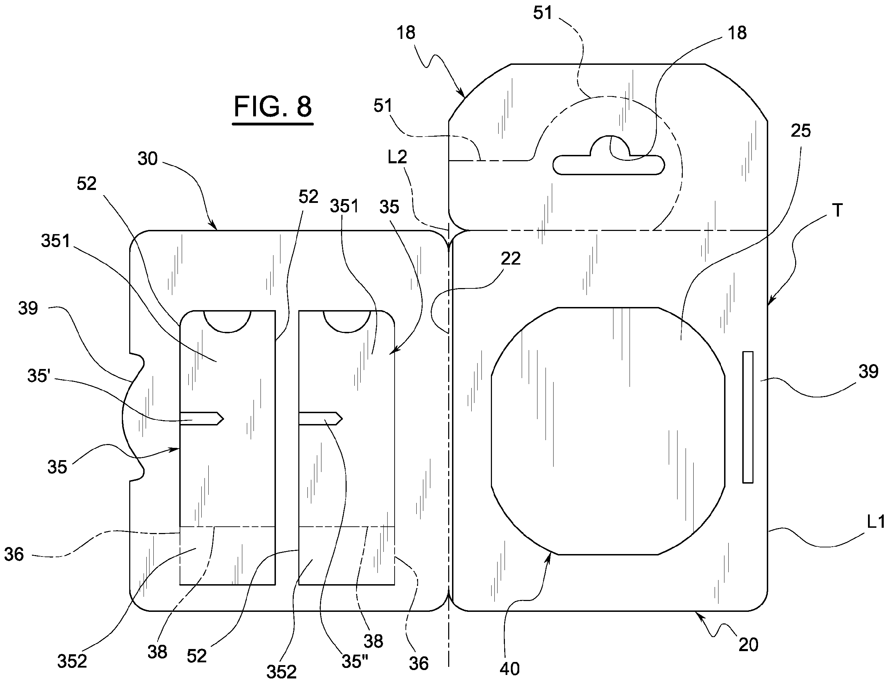

Further, in the second embodiment, the wings 35 each possess an upper portion 351 separated from the third wall 30, of which they are a portion, by means of a cutting line, while, on the contrary, the lower portion 352 is joined to the wall 30 and is foldable with respect thereto along the vertical fold edge 36 (illustrated with dot-and-dash line in figure 8). Therefore each wing 35 is joined to the wall 30 only along the line 36, while the remaining part of the perimeter of the wing is separated from the wall 30.

The upper portion 351 not only rotates with respect to the wall 30 about the vertical line 36 but is also foldable about a horizontal line 38, which separates the two portions 351 and 352 from one another; further, the upper portion 351 includes a cut 35' and respectively 35" on a vertical side in an intermediate position.

In detail, the cut 35' is located on the vertical side of a wing 35, more distant from the wall 30, while on the other wing 35 the cut 35" is performed on the vertical side adjacent to the wall 30.

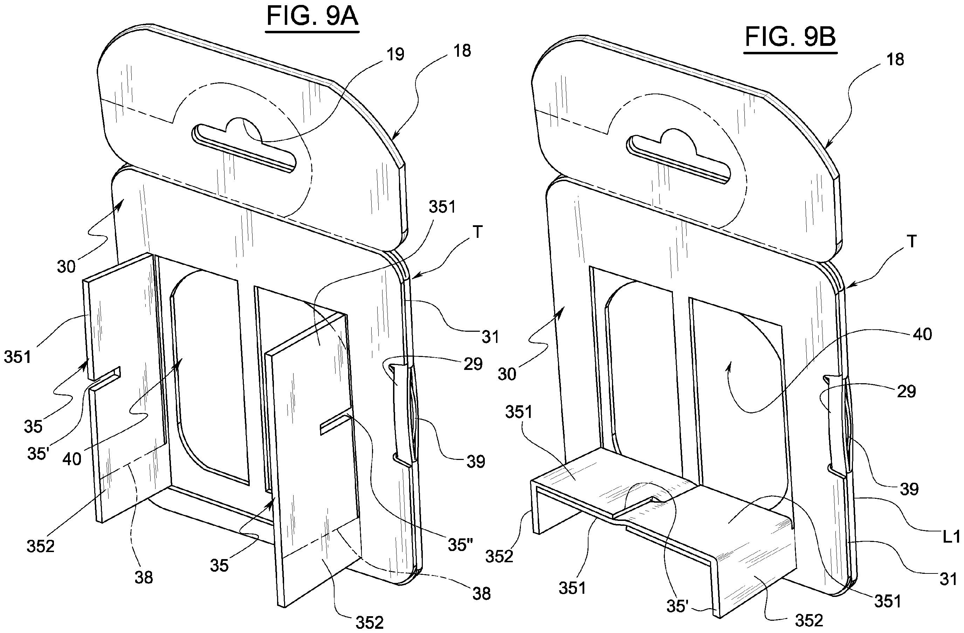

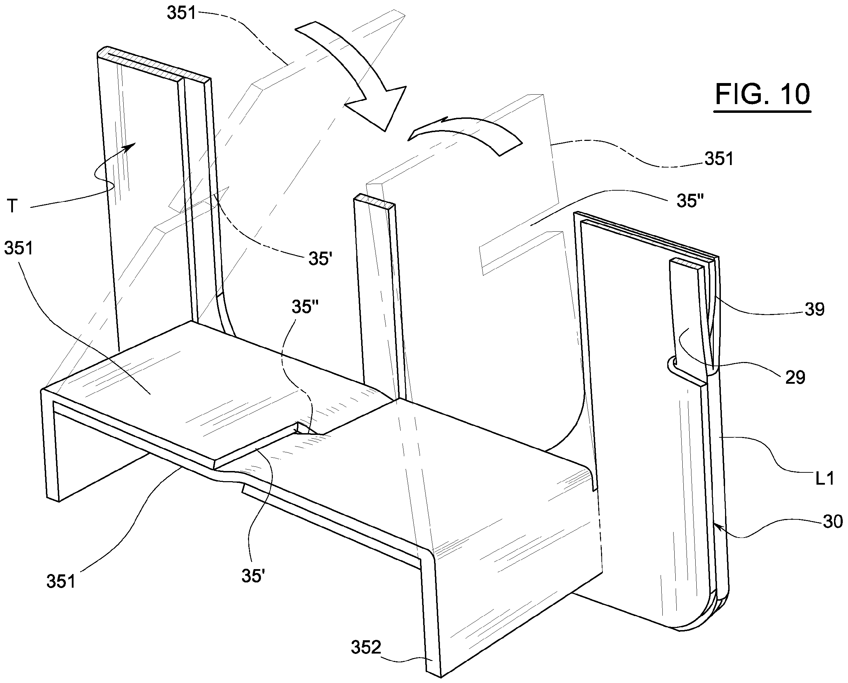

When the third wall 30 is in the described first configuration and as the wings are folded (about the line 36) at a substantially right-angle with respect to the pouch (as illustrated in figure 9A), the upper portions 351 cab be folded at a right angle, about the respective fold lines 38, and brought into a horizontal position; further they can be reciprocally jointed by braiding by a lateral edge thereof inserted in the cut 35', 35" of the other portion 351, as illustrated in figure 10.

With these characteristics, the two lower portions of wing 352, which function as rest feet for supporting the pouch T in an erect position, are constrained to one another by the upper portions 351 and therefore are maintained in a sufficiently stationary position, perpendicular to the wall 30.

Further, when the upper portions 351 of the wings 35 are folded about the fold lines 38, the portions 351 are arranged below the third openings 37 which they themselves form in the wall 30.

Therefore, in a case where the cartridge 40 and the contents thereof are sufficiently transparent, the wings 35 are not visible by the observer positioned frontally of the pack-cartridge system, which tendentially enhances the aesthetic appeal thereof.

In the third embodiment, illustrated in figures 11 and 12, the wall 10 and the wall 20 are foldably hinge-connected to one another, along a first fold line L1 located at a respective lower horizontal fold side of the card, which defines the lower end side common to both the two walls 10 and 20; at the opposite end, the upper horizontal sides of the two walls 10 and 20 are instead separate; these two upper sides can be distanced from one another and define an access side of the pouch T, through which it is possible to extract/introduce the cartridge 40, internally of the pouch with the cup 42 which projects out through the opening 15.

The wall 10 exhibits an upper portion 18, which extends in a relatively significant amount upwards with respect to the pack wall which defines the pouch T and with respect to the wall 20. An opening 19' is fashioned in this portion 18 which enables the system to be hung from a hook.

The first wall 10 and the third wall 30 are foldably hinge-connected to one another, along a second fold line L2 which is located at the upper horizontal end side of the wall 10; the third wall 30 covers, with the lower portion thereof, the access side Lt of the pouch T when it is in the first configuration, while it leaves the access side Lt accessible when it is rotated away from the second wall 20, in the above-mentioned second configuration.

The third wall 30 superposes, with the lower horizontal edge 33' thereof, on the upper horizontal edge 23' of the second wall 20 when it is in the first configuration, and is removably connectable thereto. In detail, the upper edge 33' has a projecting tongue 61 which projects outwards, which snugly inserts, with a sufficient constraint, in an upper opening 62 realised on the edge 23'; when the tongue 62 is inserted in the opening 62, the third wall 30 (first configuration), closes the access side of the pouch.

The third embodiment differs from the first and the second embodiment as the wings 35 are fashioned as portions of the second wall 20, rather than portions of the third wall 30; the opening of the second wall 20 is defined by the openings 37 which derive by folding the wings 35 vertically with respect to the pouch T.

Obviously numerous modifications of a practical-applicational nature can be made to the invention, without its forsaking the scope of the inventive idea as claimed below.

## Claims

1. A multi-use packing system with a perfumed cartridge, comprising
- a pack having three walls made of card material, hinge-connected two-by-two according to fold lines (L1, L2) in end sides of the walls,
- a replacement cartridge (40),
wherein the pack comprises:
a flat pouch (T) formed by a first wall (10) and by a second wall (20), which are superposed and fixed to one another in a substantially planar structure, the first wall (10) has at least a first opening (15),
the replacement cartridge (40) is arranged removably in the pouch (T), at least partly exposed in the first opening (19),
the second wall (20) having a second opening (25) which at least partly exposes a rear face of the replacement cartridge (40),
the third wall (30) being foldable with respect to the pouch (T);
- in a first configuration in which it is laid on the pouch (T) in a substantially planar state, in which the replacement cartridge (40) cannot be removed from the pouch; and
- in a second configuration in which the third wall (30) is rotated distant from the pouch (T) in a state that enables a user to remove the replacement cartridge (40) from the pouch (T), **characterized by** that:
at least a wing (35) being provided on the second wall (20) or on the third wall (30), which wing (35) is hinge-connected to the wall (20, 30) foldably about a respective vertical fold edge (36), where the wing (35) supports the pouch (T) in a stable erect position as a display frame, when, the third wall (30) being in the first configuration, the wing (35) is folded substantially by a right angle with respect to the pouch (T),
the pouch (T) has an open access side (Lt) for introduction/extraction of the replacement cartridge (40), and
the third wall (30) covers the access side (Lt) when it is in the first configuration, and leaves the access side (Lt) accessible when it is rotated away from the second wall (20), in the second configuration.

2. The packing system of claim 1, wherein the third wall (30) at least partly superposes the second wall (20) when it is in the first configuration, and is removably connectable thereto.

3. The packing system of claim 2 , wherein the third wall (30) comprises a projecting tongue (39) able to be snugly and removably inserted below a strip, or in an opening, afforded in the second wall (20), for retaining the tongue (39), and for maintaining the third wall (30) in the first configuration.

4. The packing system of any one of the preceding claims, wherein the first wall (10) and the third wall (30) are hinge-connected to one another along a second vertical folding edge (L2) located at an end side of the two walls (10, 30), and wherein the access side (Lt) of the pouch is vertically arranged at the second folding edge (L2) and is parallel thereto.

5. The packing system of claim 1, wherein the wings (35) are fashioned as a portion of the second or third wall (20, 30), partially separated from the wall and hinge-connected thereto foldably about vertical fold lines (36), and wherein the wings (35) open at least a third opening (37) in the wall (20, 30) of which they are portions, when they are folded by an angle with respect thereto.

6. The packing system of claims 4 and 5, wherein the third wall substantially entirely covers the opening (25) of the second wall, wherein the wings (35) are fashioned in the third wall, in such a position that the third opening at least partially superposes on the second opening of the second wall.

7. The packing system of claim 4 and 5, wherein the wings (35) each possess an upper portion (351), separate from the wall of which the wings (35) are a portion, which is foldable about a horizontal line (38), and exhibits a cut (35') and respective a further cut (35"), in an intermediate position, wherein, with the third wall (30) in the first configuration, and the wings (35) being folded at a substantial right-angle with respect to the pouch (T), the upper portions (351) can be folded into a horizontal position about the respective fold lines (38), and can be fitted reciprocally by a lateral edge thereof inserted in the cut (35', 35") of the other portion (351).

8. The packing system of claim 7, wherein, when the upper walls (351) of the wings (35) are folded about the fold lines (38), the portions (351) are arranged below the third opening.

9. The packing system of claim 1, wherein a pre-cut line (51) of the walls (10, 20) is present in the upper part of the pouch (T), such that, by removing the zone delimited by the line, an upper engaging element (17) can be obtained for hanging the system to a rear-view mirror of motor vehicles.

## Patentansprüche

1. Mehrfach verwendbares Packungssystem mit einem Dufteinsatz, umfassend:
- eine Packung mit drei Wänden, die aus einem Kartonmaterial bestehen und von denen jeweils zwei entsprechend den Falzlinien (L1, L2) an Endseiten der Wände durch ein Gelenk verbunden sind,
- einen Austauscheinsatz (40),
wobei die Packung umfasst:
eine flache Tasche (T), die von einer ersten Wand (10) und von einer zweiten Wand (20) gebildet wird, die übereinander liegen und in einer im Wesentlichen ebenen Struktur aneinander befestigt sind,
wobei die erste Wand (10) mindestens eine erste Öffnung (15) aufweist,
wobei der Austauscheinsatz (40) entnehmbar in der Tasche (T) angeordnet ist, mindestens teilweise in der ersten Öffnung (19) freiliegend,
die zweite Wand (20) eine zweite Öffnung (25) aufweist, die mindestens teilweise eine Rückseite des Austauscheinsatzes (40) freilegt,
die dritte Wand (30) in Bezug auf die Tasche (T) faltbar ist,
- in einer ersten Konfiguration, in der sie auf die Tasche (T) in einen im Wesentlichen ebenen Zustand gelegt ist, in dem der Austauscheinsatz (40) nicht aus der Tasche entnommen werden kann, und
- in einer zweiten Konfiguration, in der die dritte Wand (30) auf Distanz von der Tasche (T) in einen Zustand gedreht ist, der es einem Nutzer ermöglicht, den Austauscheinsatz (40) aus der Tasche (T) zu entnehmen,
**dadurch gekennzeichnet, dass**:
mindestens ein Flügel (35) an der zweiten Wand (20) oder an der dritten Wand (30) bereitgestellt ist, wobei der Flügel (35) durch ein Gelenk mit der Wand (20, 30) verbunden ist, faltbar um eine entsprechende vertikale Falzachse (36), wo der Flügel (35) die Tasche (T) in einer stabilen aufrechten Position als Schaurahmen stützt, wenn der Flügel (35) im Wesentlichen um einen rechten Winkel in Bezug auf die Tasche (T) gefaltet ist, während sich die dritte Wand (30) in der ersten Konfiguration befindet,
die Tasche (T) eine offene Zugangsseite (Lt) zum Einführen/Herausnehmen des Austauscheinsatzes (40) aufweist und
die dritte Wand (30) die Zugangsseite (Lt) bedeckt, wenn sie sich in der ersten Konfiguration befindet, und die Zugangsseite (Lt) zugänglich belässt, wenn sie in der zweiten Konfiguration von der zweiten Wand (20) weg gedreht ist.

2. Packungssystem nach Anspruch 1, wobei die dritte Wand (30) mindestens teilweise über der zweiten Wand (20) liegt, wenn sie sich in der ersten Konfiguration befindet, und entnehmbar mit dieser verbunden werden kann.

3. Packungssystem nach Anspruch 2, wobei die dritte Wand (30) eine vorstehende Zunge (39) umfasst, die gut passend und entnehmbar unterhalb eines Streifens oder in eine Öffnung, der/die in der zweiten Wand (20) vorhanden ist, eingeführt werden kann, um die Zunge (39) festzuhalten und um die dritte Wand (30) in der ersten Konfiguration zu halten.

4. Packungssystem nach einem der vorhergehenden Ansprüche, wobei die erste Wand (10) und die dritte Wand (30) entlang einer zweiten vertikalen Falzkante (L2), die sich an einer Endseite der beiden Wände (10, 20) befindet, durch ein Gelenk miteinander verbunden sind und wobei die Zugangsseite (Lt) der Tasche vertikal an der zweiten Falzkante (L2) angeordnet ist und parallel zu dieser liegt.

5. Packungssystem nach Anspruch 1, wobei die Flügel (35) als Abschnitt der zweiten oder dritten Wand (20, 30) gefertigt sind, teilweise getrennt von der Wand und durch ein Gelenk faltbar um vertikale Falzlinien (36) mit dieser verbunden, und wobei die Flügel (35) in der Wand (20, 30), deren Abschnitt sie sind, mindestens eine dritte Öffnung (37) öffnen, wenn sie um einen Winkel in Bezug auf die Wand gefaltet werden.

6. Packungssystem nach Anspruch 4 und 5, wobei die dritte Wand die Öffnung (25) der zweiten Wand im Wesentlichen vollständig bedeckt, wobei die Flügel (35) in der dritten Wand gefertigt sind, an einer derartigen Position, dass die dritte Öffnung mindestens teilweise über der zweiten Öffnung der zweiten Wand liegt.

7. Packungssystem nach Anspruch 4 und 5, wobei die Flügel (36) jeweils einen oberen Abschnitt (351) aufweisen - getrennt von der Wand, von der die Flügel (35) ein Abschnitt sind -, der um eine horizontale Linie (38) faltbar ist und einen Schnitt (35') und einen entsprechenden weiteren Schnitt (35") an einer Zwischenposition aufweist, wobei, wenn sich die dritte Wand (30) in der ersten Konfiguration befindet und die Flügel (35) in einem im Wesentlichen rechten Winkel in Bezug auf die Tasche (T) gefaltet sind, die oberen Abschnitte (351) um die entsprechenden Falzlinien (38) in eine horizontale Position gefaltet werden können und durch eine ihrer Seitenkante, die in den Schnitt (35', 35") des anderen Abschnitts (351) eingeführt wird, aneinander befestigt werden können.

8. Packungssystem nach Anspruch 7, wobei die Abschnitte (351) unterhalb der dritten Öffnung angeordnet sind, wenn die oberen Wände (351) der Flügel (35) um die Falzlinien (38) gefaltet sind.

9. Packungssystem nach Anspruch 1, wobei die vorgestanzte Linie (51) der Wände (10, 20) im oberen Teil der Tasche (T) vorhanden ist, so dass durch Entnehmen der durch die Linie umgrenzten Zone ein oberes Eingriffselement (17) erzielt werden kann, um das System an einen Innenrückspiegel von Kraftfahrzeugen zu hängen.

## Revendications

1. Système d'emballage multi-usage avec une cartouche parfumée, comprenant
- un emballage ayant trois parois réalisées en matériau de carte, reliées par charnière deux à deux suivant des lignes de pliage (L1, L2) dans des côtés d'extrémité des parois,
- une cartouche de rechange (40),
dans lequel l'emballage comprend :
une pochette plate (T) formée par une première paroi (10) et par une deuxième paroi (20), qui sont superposées et fixées l'une à l'autre dans une structure sensiblement plane, la première paroi (10) a au moins une première ouverture (15),
la cartouche de rechange (40) est agencée de manière amovible dans la pochette (T), au moins partiellement exposée dans la première ouverture (19),
la deuxième paroi (20) ayant une deuxième ouverture (25) qui expose au moins partiellement une face arrière de la cartouche de rechange (40),
la troisième paroi (30) étant repliable par rapport à la pochette (T);
- dans une première configuration dans laquelle elle repose sur la pochette (T) dans un état sensiblement plan, dans lequel la cartouche de rechange (40) ne peut pas être retiré de la pochette ; et
- dans une deuxième configuration dans laquelle la troisième paroi (30) est tournée loin de la pochette (T) dans un état qui permet à un utilisateur de retirer la cartouche de rechange (40) de la pochette (T),
**caractérisé en ce que** :
au moins une aile (35) étant disposée sur la deuxième paroi (20) ou sur la troisième paroi (30), laquelle aile (35) est reliée par charnière à la paroi (20, 30) de manière repliable autour d'un bord de pliage vertical respectif (36), où l'aile (35) supporte la pochette (T) dans une position dressée stable comme un cadre d'affichage, quand, la troisième paroi (30) étant dans la première configuration, l'aile (35) est pliée sensiblement à angle droit par rapport à la pochette (T),
la pochette (T) a un côté d'accès ouvert (Lt) pour l'introduction/l'extraction de la cartouche de rechange (40), et
la troisième paroi (30) couvre le côté d'accès (Lt) quand elle est dans la première configuration, et laisse le côté d'accès (Lt) accessible quand elle est éloignée par rotation de la deuxième paroi (20), dans la deuxième configuration.

2. Système d'emballage selon la revendication 1, dans lequel la troisième paroi (30) se superpose au moins partiellement à la deuxième paroi (20) quand elle est dans la première configuration, et peut être connectée de manière amovible à celle-ci.

3. Système d'emballage selon la revendication 2, dans lequel la troisième paroi (30) comprend une languette saillante (39) adaptée pour être insérée étroitement et de manière amovible sous une bande, ou dans une ouverture, prévue dans la deuxième paroi (20), pour retenir la languette (39), et pour maintenir la troisième paroi (30) dans la première configuration.

4. Système d'emballage selon l'une quelconque des revendications précédentes, dans lequel la première paroi (10) et la troisième paroi (30) sont reliées par charnière l'une à l'autre le long d'un deuxième bord de pliage vertical (L2) situé au niveau d'un côté d'extrémité des deux parois (10, 30), et dans lequel le côté d'accès (Lt) de la pochette est disposé verticalement au niveau du deuxième bord de pliage (L2) et est parallèle à celui-ci.

5. Système d'emballage selon la revendication 1, dans lequel les ailes (35) sont façonnées comme une portion de la deuxième ou troisième paroi (20, 30), partiellement séparées de la paroi et reliées par charnière à celle-ci de manière repliable autour de lignes de pliage verticales (36), et dans lequel les ailes (35) s'ouvrent au niveau d'au moins une troisième ouverture (37) dans la paroi (20, 30) dont elles sont des portions, quand elles sont pliées à un certain angle par rapport à celle-ci.

6. Système d'emballage selon les revendications 4 et 5, dans lequel la troisième paroi couvre sensiblement entièrement l'ouverture (25) de la deuxième paroi, dans lequel les ailes (35) sont façonnées dans la troisième paroi, dans une position telle que la troisième ouverture se superpose au moins partiellement à la deuxième ouverture de la deuxième paroi.

7. Système d'emballage selon les revendications 4 et 5, dans lequel les ailes (35) possèdent chacune une portion supérieure (351), séparée de la paroi dont les ailes (35) sont une portion, qui est pliable autour d'une ligne horizontale (38), et présente une découpe (35') et une autre découpe respective (35"), dans une position intermédiaire, dans lequel, avec la troisième paroi (30) dans la première configuration, et les ailes (35) étant repliée sensiblement à angle droit par rapport à la pochette (T), les portions supérieures (351) puissent être pliées dans une position horizontale autour des lignes de pliage respectives (38), et puissent être assemblées réciproquement par un bord latéral de celles-ci inséré dans la découpe (35', 35") de l'autre portion (351).

8. Système d'emballage selon la revendication 7, dans lequel, quand les parois supérieures (351) des ailes (35) sont repliées autour des lignes de pliage (38), les portions (351) sont disposées sous la troisième ouverture.

9. Système d'emballage selon la revendication 1, dans lequel une ligne de pré-coupe (51) des parois (10, 20) est présente dans la partie supérieure de la pochette (T), de manière que, en enlevant la zone délimitée par la ligne, un élément d'engagement supérieur (17) puisse être obtenu pour suspendre le système à un rétroviseur de véhicules à moteur.
